# EUROPEAN PATENT APPLICATION

(11) **EP 3 178 824 A1**
(43) Date of publication of application: **14.06.2017**
(21) Application number: 16382592.0
(22) Date of filing: 07.12.2016
(51) Int. Cl.: C07D 498/18

(54) **PROCESS FOR PREPARING PIMECROLIMUS**

(30) Priority: 08.12.2015 CN 201510893907; 02.06.2016 CN 201610384697
(71) Applicant: Medichem, S.A., 08970 Sant Joan Despi, Barcelona (ES); BrightGene Bio-Medical Technology Co., Ltd., Suzhou Industrial Park Suzhou Jiangsu 215123 (CN)
(72) Inventor: JIANDONG, Yuan, Suzhou, Jiangsu 215123 (CN); YANGQING, Huang, Suzhou, Jiangsu 215123 (CN); JIANWEN, Chi, Suzhou, Jiangsu 215123 (CN); LIANG, Ge, Suzhou, Jiangsu 215123 (CN); CARRERAS I VILAGRAN, Marçal, E-08029 Barcelona (ES); PUIG SERRANO, Jordi, E-17199 Canet d'Adri - Gerona (ES)
(74) Representative: ABG Patentes, S.L.

(57) **Abstract**

The present invention is related to an improved industrially applicable process for preparing pimecrolimus from ascomycin. This process shows significantly high yields, so that purification of the pimecrolimus can be accomplished by crystallization, not requiring for example of chromatographic purifications.

## Description

### Technical field

The present invention relates to an improved process for preparing pimecrolimus.

### Background Art

Pimecrolimus is an anti-inflammatory compound derived from the macrolactam natural product ascomycin, produced by certain strains of Streptomyces. Pimecrolimus is sold in the United States under the brand name ELIDEL^{®}, and is approved for the treatment of atopic dermatitis. The systematic name of pimecrolimus is (1R,9S,12S,13R,14S,17R,18E,21S,23S,24R,25S,27R)-12-[(1E)-2-{(1R,3R,4S)-4-chloro-3-methoxycyclohexyl}-1-methylvinyl]-17-ethyl-1,14-dihydroxy-23,25-dimethoxy-13,19,21,27-tetramethyl-11,28-dioxa-4-aza-tricyclo[22.3.1.0^{4,9}]octacos-18-ene-2,3,10,16-tetraone. Pimecrolimus is the 32-epichloro derivative of ascomycin. Its empirical formula is C₄₃H₆₈ClNO₁₁, its molecular weight is 810.47 and its structural formula is as follows:

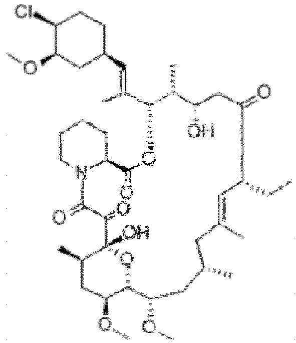

Pimecrolimus was first disclosed in the European Patent EP 0 427 680 B1. This patent discloses a method of synthesizing pimecrolimus in the form of a colourless foamy resin by means of a four-step method which comprises:
(a) protection of the two hydroxyl groups at C-24 and C-32 with t-butyldimethylsilyl ethers;
(b) deprotection of the silyl-protected hydroxyl group at C-32, while the hydroxyl group at C-24 remains protected;
(c) substitution of chlorine for the free hydroxyl group at C-32 with an inversion of configuration; and
(d) deprotection of the silyl-protected hydroxyl group at C-24.

EP 0 427 680 B1 does not disclose the yield of each step in the synthesis, but does disclose that each step is followed by the chromatographic purification of the product of that step. Accordingly, it would be expected that the overall yield of the process disclosed in EP 0 427 680 B1 is low, given the number of reaction steps and chromatographic purifications required.

EP 1 802 635 B1 discloses a one-step process for preparing pimecrolimus from ascomycin which comprises reacting ascomycin with an appropriate chlorinating agent in organic solvent, optionally in the presence of a base. According to the Examples 1 to 3 of this patent, the crude pimecrolimus needs to be purified by chromatography over silica gel giving a yield of pimecrolimus of 51.4%, 45% and 48.1% respectively. Furthermore, example 4 discloses the purification by crystallization in ethanol/water of pimecrolimus obtained in any of the examples 1 to 3 to give pimecrolimus with a yield of 87% and a purity of 98%. Therefore, the total yield to obtain pimecrolimus with a purity of 98% is not higher than 45%.

EP 1 817 317 B1 discloses a two-step process for preparing pimecrolimus, comprising dissolving ascomycin in an organic solvent, combining the solution of ascomycin with a base to obtain a reaction mixture, combining the reaction mixture with an activating reagent to obtain an activated ascomycin derivative, combining the activated derivative of ascomycin with a chloride ion source until obtaining pimecrolimus, and recovering the pimecrolimus. Lithium chloride and benzyltriethylammonium chloride are used as chloride ion source respectively in Examples 1 and 2 of EP 1 817 317 B1. In Example 1, the obtained pimecrolimus is purified by flash chromatography to give pimecrolimus with a yield of 82.6%, but no purity data is given. In Example 3, the obtained pimecrolimus in example 2 is purified by a process, which comprises a step of chromatographic purification, to give pimecrolimus with a yield of 51% and a purity of 95.75% (area by HPLC).

Thus, EP 1 81 7317 B1 discloses the use of a single source of chloride ion. It has been found that the use of a single chlorine source in accordance with the method disclosed in EP 1 817 317 B1 leads to an incomplete conversion of ascomycin to pimecrolimus, which implies lower yields. Moreover, the presence of ascomycin in the obtained pimecrolimus increases the difficulty in the purification of pimecrolimus, making inevitable the use of chromatographic purifications.

EP 2 432 791 B1 discloses four-step and five-step processes for preparing pimecrolimus, characterized in that it comprises an enzymatic acylation step of ascomycin and an enzymatic alcoholysis step with lipase from *Candida antarctica.* The overall yields are described as about 30% for the four-step process and as about 13% for the five-step process. Both processes involve chromatographic purifications.

Therefore, there is still the need of a simple and industrially applicable process for the preparation of pure pimecrolimus with a reduced number of steps and with higher yields and purity. The present invention provides such improved process.

### Brief disclosure of the figures

Figure 1 shows the purity of pimecrolimus prepared by the method of Example 1, wherein the retention time of pimecrolimus is 37.70 min, the peak area% is 59.57%, the retention time of its tautomer is 35.66 min, the peak area% is 1.12%, namely pimecrolimus peak area% is 60.69%.
Figure 2 shows the purity of pimecrolimus prepared by the method of Example 2, wherein the retention time of pimecrolimus is 37.31 min, the peak area% is 63.39%, the retention time of its tautomer is 35.56 min, the peak area% is 1.01%, namely pimecrolimus peak area% is 64.40%.
Figure 3 shows the purity of pimecrolimus prepared by the method of Example 3, wherein the retention time of pimecrolimus is 37.27 min, the peak area% is 78.50%, the retention time of its tautomer is 35.57 min, the peak area% is 1.20%, namely pimecrolimus peak area% is 79.70%.
Figure 4 shows the purity of pimecrolimus prepared by the method of Example 5, wherein the retention time of pimecrolimus is 35.54 min, the peak area% is 96.79%, the retention time of its tautomer is 33.58 min, the peak area % is 2.23%, namely pimecrolimus peak area% is 99.02%.
Figure 5 shows the purity of pimecrolimus prepared by the method of Example 7, wherein the retention time of pimecrolimus is 35.59 min, the peak area% is 97.15%, the retention time of its tautomer is 33.59 min, the peak area% is 1.86%, namely pimecrolimus peak area% is 99.01%.
Figure 6 shows the purity of pimecrolimus prepared by the method of Example 9, wherein the retention time of pimecrolimus is 35.51 min, the peak area% is 97.04%, the retention time of its tautomer is 33.57 min, the peak area% is 2.38%, namely pimecrolimus peak area% is 99.42%.

### Disclosure of the Invention

The present invention provides a process for preparing pimecrolimus which comprises:
a) dissolving ascomycin in an organic solvent;
b) activating ascomycin at C-32 by combining ascomycin with an organic or inorganic base and an activating reagent to obtain an activated ascomycin derivative;
c) reacting the activated ascomycin derivative with two chloride ion sources A and B to obtain pimecrolimus; and
d) recovering the obtained pimecrolimus,
characterized in that the chloride ion source A is selected from the group consisting of: lithium chloride, sodium chloride, potassium chloride, magnesium chloride, calcium chloride, aluminum chloride, iron(II) chloride, iron(III) chloride, ammonium chloride, and mixtures thereof; and the chloride ion source B is selected from the group consisting of quaternary phosphonium chlorides, quaternary ammonium chlorides such as tetrabutylammonium chloride and benzyltriethylammonium chloride and hydrochlorides of organic bases selected from the group consisting of triethylamine, diisopropylethylamine (DIPEA), N-methylmorpholine, N,N-dimethylaniline, pyridine and substituted pyridine derivatives such as 2,6-lutidine, 2,4,6-collidine and 4-dimethylaminopyridine, and mixtures thereof.

The authors of the present invention have found that yields in the reaction between the activated ascomycin and the chloride ions surprisingly increase when a combination of two chloride ion sources, herein referred to as chloride ion source A and chloride ion source B, is used instead of a single chloride ion source. Namely, for the same molar ratio of chloride ions versus ascomycin, the yields are significantly higher when two chloride ion sources A and B are used instead of a single chloride ion source (either A or B). This increase in the yield allows to recover a crude pimecrolimus with a higher purity, and in consequence the purification of this crude pimecrolimus is easier, e.g., not requiring chromatographic purification or requiring less steps of purification.

In one preferred embodiment of the present invention, the organic solvent used for dissolving ascomycin is selected from the group consisting of dichloromethane, chloroform, diethyl ether, diisopropyl ether, tetrahydrofuran, 2-methyltetrahydrofuran, methyl t-butyl ether, toluene, xylene, hexane, heptane, cyclohexane, methylcyclohexane, acetonitrile and mixtures thereof, preferably dichloromethane, toluene, acetonitrile or mixtures thereof.

Preferably, the organic solvent used for dissolving ascomycin is anhydrous. Alternatively, the obtained solution of ascomycin can be anhydrified, e.g., by using molecular sieves.

In another preferred embodiment of the present invention, the resulting ascomycin solution is combined with the activating agent to obtain the activated ascomycin derivative at a temperature lower than 0°C, preferably lower than -20°C, more preferably between -35°C and -30°C. This provides a particularly advantageous process, in particular leading to a highly pure product with better yields.

The organic or inorganic base used in step b) of the process of the present invention can be added dropwise, in parts, or all at once.

In a preferred embodiment of the present invention, the base used in step b) is an organic base selected from the group consisting of triethylamine, diisopropylethylamine (DIPEA), N-methylmorpholine, N,N-dimethylaniline, pyridine and substituted pyridine derivatives such as 2,6-lutidine, 2,4,6-collidine and 4-dimethylaminopyridine, preferably diisopropylethylamine (DIPEA), 2,6-lutidine, 2,4,6-collidine or mixtures thereof.

The organic or inorganic base used in step b) of the process of the present invention can be added before the addition of the activating agent or together with the activating agent. Preferably, the organic or inorganic base is added before the addition of the activating agent.

The organic or inorganic base used in step b) of the process of the present invention may be added alone or in solution, where the solvent is preferably selected from the group consisting of: dichloromethane, chloroform, diethyl ether, diisopropyl ether, tetrahydrofuran, 2-methyltetrahydrofuran, methyl t-butyl ether, toluene, xylene, hexane, heptane, cyclohexane, methylcyclohexane and mixtures thereof.

The organic or inorganic base used in step b) of the process of the present invention is preferably used in an amount of about 1 to about 6 molar equivalents with respect to ascomycin, more preferably used in an amount of about 3 to about 4 molar equivalents with respect to ascomycin.

Following or with the addition of the organic or the inorganic base, the reaction mixture is combined with the activating agent which can be added over the reaction mixture dropwise, in parts, or all at once. Preferably, the resulting solution is stirred at a temperature lower than 0°C, preferably lower than -20°C, more preferably between - 35°C and -30°C. This provides a particularly advantageous process, in particular leading to a highly pure product with better yields. Preferably, the progress of the reaction is monitored, e.g., by thin layer chromatography (TLC), until the reaction is completed or nearly completed.

In another preferred embodiment of the present invention, the activated ascomycin derivative is a sulfonate ester thereof selected from tosylate, mesylate and triflate, preferably triflate.

In another preferred embodiment of the present invention, the activating agent is selected from the group consisting of fluorosulfonic anhydride, fluorosulfonyl chloride, trifluoromethanesulfonic anhydride, trifluoromethanesulfonyl chloride, methanesulfonic anhydride, methanesulfonyl chloride, phenylmethanesulfonic anhydride, phenylmethanesulfonyl chloride, p-toluenesulfonic anhydride, p-toluenesulfonyl chloride, benzenesulfonic anhydride, and benzenesulfonyl chloride, preferably the activating agent is trifluoromethanesulfonic anhydride.

The activating agent can be added alone or in solution, where the solvent is selected from the same group of solvents that is used to form the solution with the base, as described above.

The time required for the complete or near complete conversion of the ascomycin to the corresponding activated ascomycin can vary somewhat, depending upon the reaction conditions, such as the temperature, and the solvent, base, and activating agent utilized. Those skilled in the art will understand how to monitor the reaction, e.g., by TLC, at appropriate time intervals, depending on the conditions chosen.

Once the activated ascomycin derivative is obtained, the reaction mixture is then combined with the two chloride ion sources A and B. The two chloride ion sources A and B can be added over the reaction mixture together simultaneously or in sequence, alone or in solution. Useful solvents for the chloride ion sources include those discussed above for the organic or inorganic base and the activating agent.

In one preferred embodiment, during the addition of the chloride ion sources A and B, the reaction mixture is maintained at a temperature preferably lower than 20°C, more preferably lower than 10°C, even more preferably around 5°C. This provides a particularly advantageous process, in particular leading to a highly pure product with better yields.

In another preferred embodiment, during the addition of the chloride ion sources A and B, the reaction mixture is maintained at a temperature preferably lower than 0°C, more preferably equal or lower than -10°C, even more preferably between -35°C and -10°C. This provides a particularly advantageous process, in particular leading to a highly pure product with better yields.

The chloride ion source A is selected from the group consisting of: lithium chloride, sodium chloride, potassium chloride, magnesium chloride, calcium chloride, aluminum chloride, iron(II) chloride, iron(III) chloride, ammonium chloride, and mixtures thereof; and the chloride ion source B is selected from the group consisting of quaternary phosphonium chlorides, quaternary ammonium chlorides such as tetrabutylammonium chloride and benzyltriethylammonium chloride and hydrochlorides of organic bases selected from the group consisting of triethylamine, diisopropylethylamine (DIPEA), N-methyl-morpholine, N,N-dimethylaniline, pyridine and substituted pyridine derivatives such as 2,6-lutidine, 2,4,6-collidine and 4-dimethylaminopyridine, and mixtures thereof.

In a preferred embodiment of the present invention, the chloride ion source A is lithium chloride and the chloride ion source B is benzyltriethylammonium chloride.

In another preferred embodiment of the present invention, the molar ratio of the chloride ion source A versus the chloride ion source B is from about 5:1 to about 1:5, preferably from about 2:1 to about 1:2.

In a preferred embodiment of the present invention, the molar ratio of the chloride ion source A versus the chloride ion source B is about 1.8:1. This provides a particularly advantageous process, in particular leading to a highly pure product with better yields.

In another preferred embodiment of the present invention, the molar ratio of the chloride ion source A versus the chloride ion source B is about 1:1. This provides a particularly advantageous process, in particular leading to a highly pure product with better yields.

Preferably, the molar ratio of the sum of chloride ion source A plus the chloride ion source B versus ascomycin is higher than 1, more preferably higher than 4, even more preferably about 20.

In a preferred embodiment, once the addition of the chloride ion sources A and B is finished, the reaction mixture is preferably stirred at a temperature lower than 20°C, preferably lower than 0°C, more preferably between -10°C and 0°C, even more preferably between -5°C and 0°C. Again, the progress of the reaction is monitored to determine completion, such as by TLC. This provides a particularly advantageous process, in particular leading to a highly pure product with better yields.

In another preferred embodiment, after the chloride ion sources A and B are added, the reaction mixture is stirred at 20°C to 35°C, preferably at 20°C to 30°C. Likewise, the monitoring reaction was carried out to confirm the reaction was complete, as by TLC. This provides a particularly advantageous process, in particular leading to a highly pure product with better yields.

The time required for the disappearance of the intermediate activated ascomycin derivative will vary somewhat depending on the precise conditions used. Those skilled in the art will understand how to monitor the reaction, e.g., by TLC, at appropriate time intervals, depending on the conditions chosen.

The pimecrolimus recovering step of the process of the present invention comprises: adding water to the reaction mixture to obtain a two phase system; separating the two phase system and concentrating the organic phase to obtain a first residue. This residue is preferably in form of amorphous solid. The pimecrolimus recovering step can further comprise combining this residue with a water-immiscible organic solvent and washing the resulting solution with an aqueous acidic solution and water; collecting the organic phase and concentrating the organic phase to obtain a second residue.

Non-limiting examples of water-immiscible organic solvents that can be added to the first residue are diisopropyl ether, diethyl ether, 2-methyltetrahydrofuran, cyclopentyl methyl ether, methyl tert-butyl ether; methyl ethyl ketone, methyl isobutyl ketone, dichloromethane, chloroform, tetrachloromethane, dichloroethane, chlorobenzene or dichlorobenzene; hydrocarbon aliphatic solvents such as methylcyclohexane, cyclohexane, heptane or hexane; hydrocarbon aromatic solvents such as toluene, benzene, o-xylene, m-xylene or p-xylene or mixtures of two or more of the solvents listed. Preferably the water-immiscible organic solvents that can be added to the first residue are dichloromethane, heptane or mixtures thereof.

In a preferred embodiment, the recovered pimecrolimus is purified by means of recrystallization. The purified pimecrolimus is preferably in crystalline form, more preferably the purified pimecrolimus is crystalline pimecrolimus Form A or Form B as described in WO 99/01458 A1.

Non limiting examples of suitable solvents for the recrystallization of the pimecrolimus obtained according to the present invention are alcohols such as methanol, ethanol, isopropanol or butanol; ethers such as tetrahydrofuran, dioxane, diisopropyl ether, diethyl ether, 2-methyltetrahydrofuran, cyclopentyl methyl ether or methyl tert-butyl ether; ketones such as methyl ethyl ketone, methyl isobutyl ketone or acetone; halogenated solvents such as dichloromethane, chloroform, tetrachloromethane, dichloroethane, chlorobenzene or dichlorobenzene; polar aprotic solvents such as N,N-dimethylformamide, acetonitrile, N,N-dimethylacetamide, N-methyl-2-pyrrolidone or dimethylsulfoxide; hydrocarbon aliphatic solvents such as methylcyclohexane, cyclohexane, heptane or hexane; hydrocarbon aromatic solvents such as toluene, benzene, o-xylene, m-xylene or p-xylene or water or mixtures of two or more of the solvents listed. Particularly preferred solvents are alcohols, ketones, hydrocarbon aliphatic solvents and water or mixtures thereof. More preferred solvents are mixtures of alcohols and hydrocarbon aliphatic solvents.

Alternatively, the crude product can be purified by column chromatography.

Preferably, the purified pimecrolimus has a purity higher than 97% area by HPLC, preferably higher than 98% area by HPLC and more preferably higher than 99% area by HPLC.

The term "about" when used in the present invention preceding a number and referring to it, is meant to designate any value which lies within the range defined by the number ±10% of its value, preferably a range defined by the number ±5%, more preferably range defined by the number ±2%, still more preferably a range defined by the number ±1%. For example "about 10" should be construed as meaning within the range of 9 to 11, preferably within the range of 9.5 to 10.5, more preferably within the range of 9.8 to 10.2, and still more preferably within the range of 9.9 to 10.1.

The term "anhydrous" when used in the present invention means that the organic solvent comprises less than 1% (w/w) of water, more preferably less than 0.5% (w/w) of water, and even more preferably less than 0.1 % (w/w) of water.

The term "crystalline" as used in the present invention means that at least 20% (w/w) of the pimecrolimus obtained according to the process of the present invention is crystalline, preferably more than 50% (w/w) of the pimecrolimus obtained according to the process of the present invention is crystalline, more preferably more than 70% (w/w) of the pimecrolimus obtained according to the process of the present invention is crystalline and even more preferably more than 90% (w/w) of the pimecrolimus obtained according to the process of the present invention is crystalline.

### Experimental examples

The following examples further illustrate the invention but, of course, should not be construed as in any way limiting its scope.

### General experimental conditions:

### HPLC method used in examples 1-3, 5-9

The chromatographic separation was carried out using a Poroshell 120 EC-18, 2.7 mm (4.6 x 150 mm) column, at 60°C.

The mobile phase A was 0.1 % (V/V) phosphoric acid solution in water.

The mobile phase B was methanol.

The chromatograph was programmed as follows: Initial 0-10 min. isocratic 35% mobile phase A, 10-30 min. linear gradient to 25% mobile phase A, 30-60 min. isocratic 25% mobile phase A; 60-63 min. linear gradient to 35% mobile phase A. 63-70 min. isocratic 35% mobile phase A.

The chromatograph was equipped with a 210 nm UV detector. The flow rate was 1.0 mL/min.

Test samples were prepared by dissolving about 40 mg of sample in 10.0 mL of diluent (acetonitrile). 10 µl of the test samples were injected.

### HPLC method used in example 4

The chromatographic separation was carried out using a Poroshell 120 EC-18, 2.7 mm (4.6 x 150 mm) column, at 60°C.

The mobile phase A was 0.08% (V/V) phosphoric acid solution in water.

The mobile phase B was 0.08% (V/V) phosphoric acid solution in methanol.

The chromatograph was programmed as follows: Initial 0-2 min. isocratic 33% mobile phase A, 2-38 min. linear gradient to 27% mobile phase A, 38-50 min. isocratic 27% mobile phase A; 50-56 min. linear gradient to 33% mobile phase A. 56-68 min. isocratic 33% mobile phase A.

The chromatograph was equipped with a 210 nm UV detector. The flow rate was 1.0 mL/min.

Test samples were prepared by dissolving about 60 mg of sample in 10.0 mL of diluent (acetonitrile with 0.1% (V/V) formic acid). 10 µl of the test samples were injected.

### Example 1: Comparative example based on Example 1 of EP 1 817 317 B1 (using a chloride ion source A)

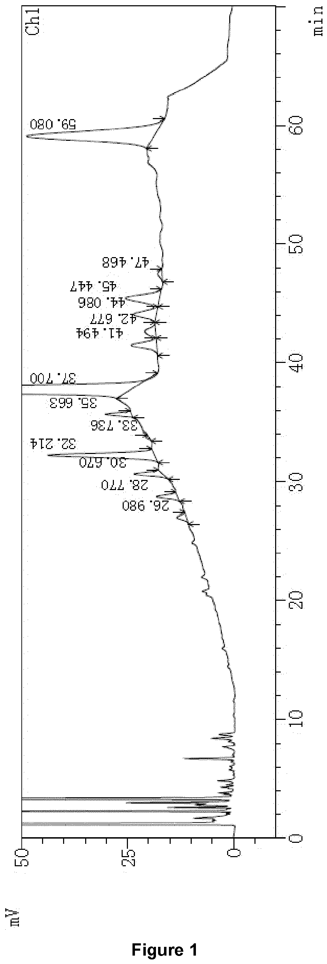

3.0 g (3.79 mmol) of ascomycin (94.7%, purity % area HPLC) were dissolved in 25 mL of anhydrous dichloromethane under nitrogen atmosphere. The solution was cooled to -15°C. Then, 24 mL of a 5% w/w solution of trifluoromethanesulfonic anhydride in anhydrous dichloromethane (in a first addition funnel) and 1.8 g of 2,6-lutidine (in a second addition funnel) were added simultaneously to the reaction mixture. Once the addition was completed, 28 g of a 10% w/w solution of LiCl (66.05 mmol, 17.4 molar equivalents with respect to ascomycin) in dichloromethane were added to the reaction mixture. Once the addition was completed, the reaction mixture was heated to about 21°C and stirred at this temperature for 4 days. The resulting reaction mixture was diluted with a mixture of 200 mL of ethyl acetate and 25 mL of water, the organic layer was extracted and evaporated to dryness.
Purity (% area, HPLC): 60.69%. HPLC spectrum is shown in Figure 1.

### Example 2: Comparative example based on Example 2 of EP 1 817 317 B1 (using a chloride ion source B)

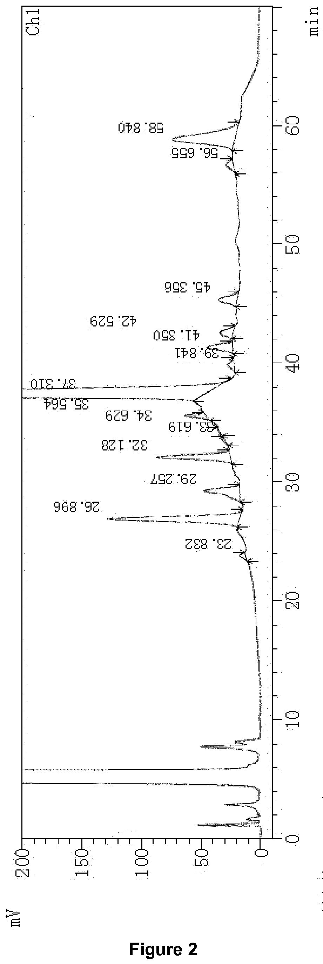

2.9 g (3.66 mmol) of ascomycin (94.7%, purity % area HPLC) were dissolved in 20 mL of toluene. The solution was concentrated to dryness at 40 °C. The obtained residue was dissolved in 23 mL of anhydrous toluene under nitrogen atmosphere, and then 28 mL of anhydrous acetonitrile were added. The resulting solution was cooled to -15°C and 1.13 g of diisopropylethylamine were added to the reaction mixture. Then, a solution of 1.39 g of trifluoromethanesulfonic anhydride in 32 mL of previously cooled (-15°C) anhydrous toluene were added to the reaction mixture. Once the addition was completed, temperature was increased to 0°C and then 30 g of a 12.5% w/w solution of benzyltriethylammonium chloride (16.46 mmol, 4.5 molar equivalents with respect to ascomycin) in anhydrous acetonitrile were added. Then, temperature was increased to about 25°C and, after stirring for 45 minutes at 24-25°C, 20 mL of water were added. After vigorous stirring, additional 20 mL of water were added, the organic layer was extracted and evaporated to dryness.
Purity (% area, HPLC): 64.40%. HPLC spectrum is shown in Figure 2. As it is shown the crude product contains a large number of benzyltriethylammonium chloride and unreacted ascomycin.

### Example 3: Preparation of pimecrolimus according to the process of the present invention

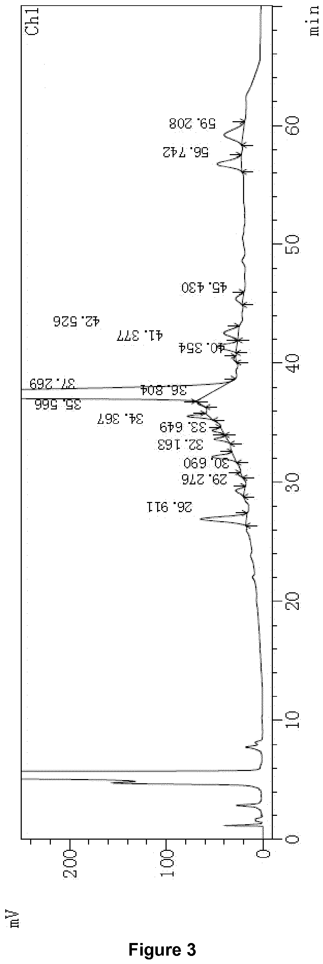

2.9 g (3.66 mmol) of ascomycin (94.7%, purity % area HPLC) were dissolved in 20 mL of toluene. The solution was concentrated to dryness at 40 °C. The obtained residue was dissolved in 23 mL of anhydrous toluene under nitrogen atmosphere, and then 28 mL of anhydrous acetonitrile were added. The resulting solution was cooled to -15°C and 1.13 g of diisopropylethylamine were added to the reaction mixture. Then, a solution of 1.39 g of trifluoromethanesulfonic anhydride in 32 mL of previously cooled (-15°C) anhydrous toluene were added to the reaction mixture. Once the addition was completed, temperature was increased to 0°C and then a solution of 1.90 g (8.34 mmol) of benzyltriethylammonium chloride and 0.34 g (8.02 mmol; i.e. total amount of chloride anion is 16.36 mmol, *i.e.* 4.5 molar equivalents with respect to ascomycin) of LiCl in 35 mL of anhydrous acetonitrile were added. Then, temperature was increased to about 25°C and, after stirring for 45 minutes at 24-25°C, 20 mL of water were added. After vigorous stirring, additional 20 mL of water were added, the organic layer was extracted and evaporated to dryness.
Purity (% area, HPLC): 79.70%. HPLC spectrum is shown in Figure 3.

### Example 4: Preparation of pimecrolimus according to the process of the present invention

100 g of ascomycin were dissolved in 600 mL of dichloromethane at a temperature between 15°C and 20°C. 17.9 g of molecular sieves were added to this solution and the mixture was stirred at 20-25°C for 2 hours. The mixture was then filtered and the solid washed with 75 mL of dichloromethane. The filtrate was collected and cooled to a temperature between -35°C and -30°C and 46.9 g of 2,6-lutidine and 39.3 g of trifluomethanesulfonic anhydride were added in sequence. The reaction mixture was stirred for 30 minutes at a temperature between about -35°C to about -30°C. Then, 52.2 g of lithium chloride and 287.9 g of benzyltriethylammonium chloride were added while keeping the reaction temperature below -10°C. The reaction mixture was heated to a temperature between -5°C and 0°C and stirred at this temperature range for 2 hours. Then, the reaction mixture was heated to 15-20°C and stirred at this temperature range for 30 minutes. After cooling to 0°C, 400 mL of water were added and the resulting mixture was stirred for 30 minutes at a temperature between 0°C and 5°C. The organic layer was extracted and evaporated to dryness to give crude pimecrolimus with a purity of about 82.60%.

200 mL of dichloromethane and 400 mL of n-heptane were added to the obtained residue and this mixture was stirred at 20-25°C for 1 hour. The resulting suspension was filtered and the solid was washed with a mixture of 200 mL of dichloromethane and 400 mL of heptane. The filtrate was collected and washed three times with 300 mL of aqueous HCl 0.5M and three times with 300 mL of water. The organic phase was collected and evaporated to dryness.
Purity (% area, HPLC): 98.59%.

### Example 5: Purification of pimecrolimus

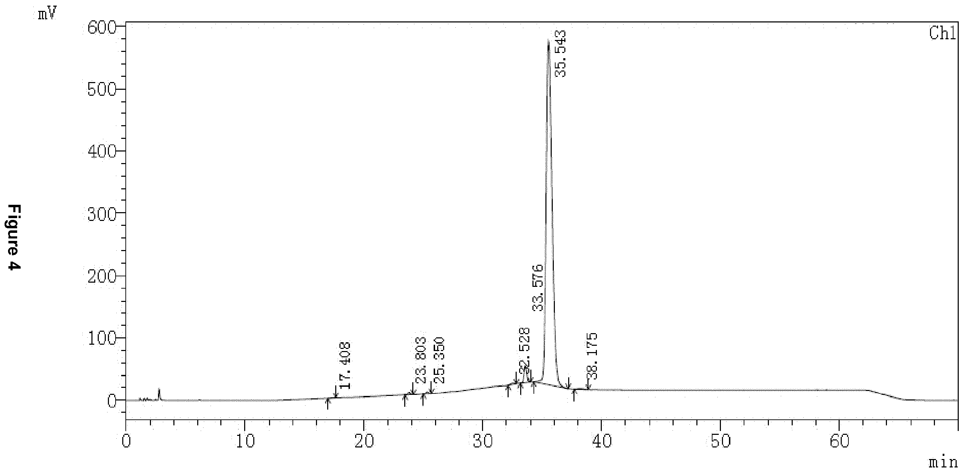

Over the residue obtained in Example 4, 390 mL of isopropanol were added and the mixture was heated to 30-35°C until complete dissolution. Then, 200 mL of water were added dropwise at a temperature between 25°C and 30°C. The mixture was stirred at 20-25°C for 2 hours. Then, the reaction mixture was cooled to 0-5°C and stirred at this temperature for 4 hours. The mixture was filtered and the solid was washed first with a mixture of 120 mL of isopropanol and 80 mL of water and secondly with 140 mL of heptane. Wet solid was dried at 35°C±5°C under vacuum to obtain 84.8 g of pimecrolimus (overall yield of 82.9% from ascomycin).
Purity (% area, HPLC): 99.02%. HPLC spectrum is shown in Figure 4.

### Example 6: Preparation of pimecrolimus according to the process of the present invention

2.9 g (3.66 mmol) of ascomycin were dissolved in 250 ml of dry methylene chloride and stirred at 20°C to 25°C for 2 hours. The resulting solution was cooled to 0°C and 1.13 g of diisopropylethylamine were added to the reaction mixture. Then, 320 ml of an anhydrous dichloromethane solution of 13.9 g of trifluoromethanesulfonic anhydride previously cooled to 0°C were added to the reaction mixture. After the completion of the addition, the reaction temperature was controlled at 0°C, and then 111.2 g (488 mmol) of benzyltriethylammonium chloride and 10.3 g (244 mmol) of LiCl dissolved in anhydrous methylene chloride (total chloride: 732 mmol, equivalent to 20 molar equivalents versus ascomycin) were added to the reaction mixture. The reaction mixture temperature was then raised to about 20°C, stirred between 20°C and 21°C for 45 minutes, and then 200 ml of water were added. After thorough stirring, 200 ml of water were added, the organic layer was extracted, and the solvent was distilled off to obtain 135 g of crude pimecrolimus with a purity of about 79%.

750 ml of ethanol were added to the crude pimecrolimus prepared above and then heated to 30-35°C until complete dissolution. 250 ml of water were added dropwise thereto at 25°C to 30°C, and the mixture was stirred at this temperature for about 2 hours. The reaction solution was then cooled to 0-5°C and stirred at this temperature for 4 hours. Then the reaction mixture was filtered, and the solid was washed with a mixture of 90 ml of ethanol and 30 ml of water and then with 100 ml of heptane. Wet solid was dried at 35°C±5°C under vacuum to give 24.3 g of pimecrolimus (total yield: 82.3% based on ascomycin).
Purity (% area, HPLC): 99.62%.

### Example 7: Preparation of pimecrolimus according to the process of the present invention

3.0 kg ascomycin were dissolved in 18 L of anhydrous dichloromethane. The resulting solution was cooled to -20°C, and 1.625 kg of a 2,6-lutidine solution were added to the reaction mixture keeping the temperature of the reaction solution equal or lower than - 20°C. A solution of 1.39 Kg of trifluoromethanesulfonic anhydride was added dropwise to the reaction mixture, keeping the temperature of the reaction mixture equal or lower than -20°C. After completion of the dropwise addition, the mixture was stirred at -20°C (± 2°C) for 1 hour. The reaction was monitored by TLC until the reaction was complete. The reaction mixture was then heated to 5°C (± 2°C), followed by the addition of 4.315 kg of benzyltriethylammonium chloride and 1.445 kg of anhydrous lithium chloride, followed by heating to 28°C (± 2°C). The reaction was monitored by TLC until the reaction was complete. Then 20 L of water were added. After thorough stirring, 20 L of water were added, the organic layer was extracted, and the solvent was distilled off to obtain 3000 g of crude pimecrolimus with a purity of about 82.1%.

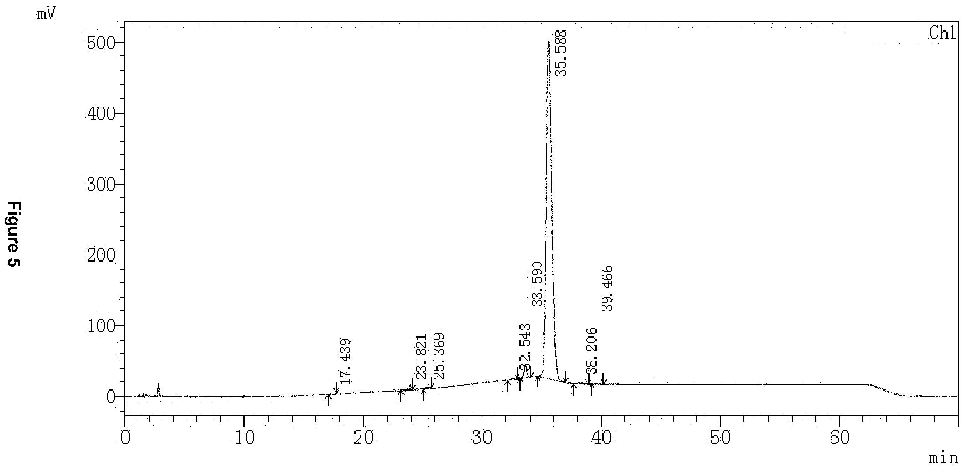

12 L of ethanol were added to the crude pimecrolimus prepared above and the mixture was then heated to 30-35°C until complete dissolution. 4 L of water were added dropwise thereto at 25°C to 30°C, and the mixture was stirred at this temperature for about 2 hours. The reaction mixture was then cooled to 0-5°C and stirred at this temperature for 4 hours. Then it was filtered, and the solid was washed with a mixture of 1.5 L of ethanol and 0.5 L of water and afterwards with 1.5 L of heptane. The resulting wet solid was dried at 35°C±5°C under vacuum to give pimecrolimus 2610 g (total yield: 87%, starting from ascomycin),
Purity (% area, HPLC): 99.01%. HPLC spectrum is shown in Figure 5.

### Example 8: Preparation of pimecrolimus according to the process of the present invention

3.0 kg ascomycin were dissolved in 18 L of anhydrous dichloromethane. The resulting solution was cooled to -20°C and 1.625 kg of a 2,6-lutidine solution was added to the reaction mixutre, keeping the temperature of the reaction mixture equal or lower than - 20°C. A solution of 1.39 Kg of trifluoromethanesulfonic anhydride was added dropwise to the reaction mixture, keeping the temperature of the reaction mixture equal or lower than -20°C. After completion of the dropwise addition, the mixture was stirred at -20°C (± 2°C) for 1 hour. The reaction was monitored by TLC until the reaction was complete. The reaction mixture was then heated to 5°C (± 2°C), followed by the addition of 4.315 kg of benzyltriethylammonium chloride and 1.445 kg of anhydrous lithium chloride, followed by heating to 28°C (± 2°C). The reaction was monitored by TLC until the reaction was complete. Then 20 L of water were added. After sufficient stirring, 20 L of water were added, the organic layer was extracted, and the solvent was distilled off to obtain crude pimecrolimus.

16 L of isopropanol were added to the crude product of pimecrolimus prepared above and then heated to 30-35°C until complete dissolution. 1.5 L of water were added dropwise thereto at 25°C to 30°C, and the mixture was stirred at this temperature for about 2 hours. The reaction solution was then cooled to 0-5°C and stirred at this temperature for 4 hours. Then filtered, and the solid was washed with a mixture of 1.5 L of isopropanol and 0.5 L of water and afterwards with 1.5 L of heptane. The resulting wet solid was dried at 35°C±5°C under vacuum to give pimecrolimus (total yield 89.1%, starting from ascomycin).
Purity (% area, HPLC): 99.89%

### Example 9: Preparation of pimecrolimus according to the process of the present invention

3.0 kg (3.79 mol) of ascomycin were dissolved in 18 L of anhydrous tetrahydrofuran. The resulting solution was cooled to -20°C, and 2.76 kg (22.74 mol) of N,N-dimethylaniline were added to the reaction mixture, keeping the temperature of the reaction mixture equal or lower than -20°C. To the reaction mixture, 0.65 kg of methanesulfonyl chloride were added dropwise, keeping the temperature of the reaction mixture equal or lower than -20°C. After completion of the dropwise addition, the mixture was stirred at -20 °C (± 2°C) for 1 hour. The reaction was monitored by TLC until the reaction was complete. The temperature of the reaction mixture was raised to 18°C (± 2°C), followed by the addition of 8.78 kg (31.6 mol) of tetrabutylammonium chloride and 0.6 kg (6.3 mol) of anhydrous magnesium chloride, followed by heating at 25°C. The reaction was monitored by TLC until the reaction was complete. Then 20 L of water were added. After thorough stirring, 20 L of water were added, the organic layer was extracted, and the solvent was distilled off to obtain 2800 g of crude pimecrolimus.

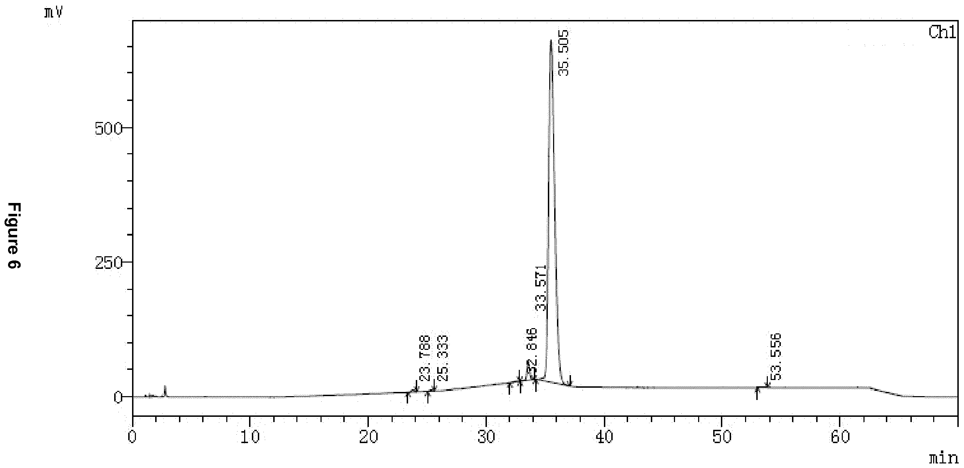

16.8 L of isopropyl alcohol were added to the crude pimecrolimus prepared above and then heated to 30-35°C until complete dissolution. 5.6 L of water were added dropwise thereto at 25°C to 30°C, and the mixture was stirred at this temperature for about 2 hours. The mixture was then cooled to 0-5°C and stirred at this temperature for 4 hours. Then filtered, and the solid was washed with a mixture of 1.5 L of isopropyl alcohol and 0.5 L of water and afterwards with 15 L of heptane. The resulting wet solid was dried at 35°C±5°C under vacuum to give pimecrolimus 2530 g (yield: 82.4%, starting from ascomycin).
Purity (% area, HPLC): 99.42%. HPLC spectrum is shown in Figure 6.

## Claims

1. Process for preparing pimecrolimus which comprises:
a) dissolving ascomycin in an organic solvent;
b) activating ascomycin at C-32 by combining ascomycin with an organic or inorganic base and an activating reagent to obtain an activated ascomycin derivative;
c) reacting the activated ascomycin derivative with two chloride ion sources A and B to obtain pimecrolimus; and
d) recovering the obtained pimecrolimus,
**characterized in that** the chloride ion source A is selected from the group consisting of: lithium chloride, sodium chloride, potassium chloride, magnesium chloride, calcium chloride, aluminum chloride, iron(II) chloride, iron(III) chloride, ammonium chloride, and mixtures thereof; and the chloride ion source B is selected from the group consisting of quaternary phosphonium chlorides, quaternary ammonium chlorides such as tetrabutylammonium chloride and benzyltriethylammonium chloride and hydrochlorides of organic bases selected from the group consisting of triethylamine, diisopropylethylamine (DIPEA), N-methylmorpholine, N,N-dimethylaniline, pyridine and substituted pyridine derivatives such as 2,6-lutidine, 2,4,6-collidine and 4-dimethylaminopyridine, and mixtures thereof.

2. The process according to claim 1, wherein the organic solvent for dissolving ascomycin is selected from the group consisting of dichloromethane, chloroform, diethyl ether, diisopropyl ether, tetrahydrofuran, 2-methyltetrahydrofuran, methyl t-butyl ether, toluene, xylene, hexane, heptane, cyclohexane, methylcyclohexane, acetonitrile and mixtures thereof, preferably dichloromethane, toluene, acetonitrile or mixtures thereof.

3. The process according to any one of claims 1 to 2, wherein the resulting ascomycin solution is combined with the activating agent to obtain the activated ascomycin derivative at a temperature lower than 0°C, preferably lower than -20°C, more preferably between -35°C and -30°C.

4. The process according to any one of claims 1 to 3, wherein the base used in step b) is an organic base selected from the group consisting of triethylamine, diisopropylethylamine (DIPEA), N-methylmorpholine, N,N-dimethylaniline, pyridine, and substituted pyridine derivatives such as 2,6-lutidine, 2,4,6-collidine and 4-dimethylaminopyridine, preferably diisopropylethylamine (DIPEA), 2,6-lutidine, 2,4,6-collidine or mixtures thereof.

5. The process according to any one of claims 1 to 4, wherein the activated ascomycin derivative is a sulfonate ester selected from tosylate, mesylate and triflate, preferably triflate.

6. The process according to any one of claims 1 to 4, wherein the activating agent is selected from the group consisting of fluorosulfonic anhydride, fluorosulfonyl chloride, trifluoromethanesulfonic anhydride, trifluoromethanesulfonyl chloride, methanesulfonic anhydride, methanesulfonyl chloride, phenylmethanesulfonic anhydride, phenylmethanesulfonyl chloride, p-toluenesulfonic anhydride, p-toluenesulfonyl chloride, benzenesulfonic anhydride, and benzenesulfonyl chloride, preferably trifluoromethanesulfonic anhydride.

7. The process according to any one of claims 1-6, wherein the quaternary ammonium chloride is selected from the group consisting of tetrabutylammonium chloride and benzyltriethylammonium chloride; and the substituted pyridine derivative is selected from the group consisting of 2,6-lutidine, 2,4,6-trimethylpyridine and 4-dimethylaminopyridine.

8. The process according to any one of claims 1 to 7, wherein the chloride ion source A is lithium chloride and the chloride ion source B is benzyltriethylammonium chloride.

9. The process according to any one of claims 1 to 8, wherein the molar ratio of the chloride ion source A versus the chloride ion source B is from about 5:1 to about 1:5, preferably from about 2:1 to about 1:2, more preferably about 1.8:1 or 1:1.

10. The process according to any one of claims 1-9, wherein during the addition of the chloride ion sources A and B, the reaction mixture is maintained at a temperature lower than 0°C, preferably equal or lower than -10°C, more preferably between -35°C and - 10°C or at a temperature lower than 20°C, preferably lower than 10°C, more preferably around 5°C.

11. The process according to any one of claims 1 to 10, wherein the reaction mixture after the addition of the two chloride sources A and B is stirred at a temperature lower than 20°C, preferably lower than 0°C, more preferably between -10°C and 0°C, even more preferably between -5°C and 0°C.

12. The process according to any one of claims 1 to 10, wherein the reaction mixture after the addition of the two chloride sources A and B is stirred at a temperature from 20°C to 35°C, preferably from 20°C to 30°C.

13. The process according to any one of claims 1-12, wherein the molar ratio of the sum of chloride ion source A plus the chloride ion source B versus ascomycin is higher than 1, more preferably higher than 4, even more preferably about 20.

14. The process according to any one of claims 1 to 13, further comprising purifying the obtained pimecrolimus by recrystallization.
